# EUROPÄISCHE PATENTSCHRIFT

(11) **EP 0 340 472 B1**
(45) Veröffentlichungstag und Bekanntmachung des Hinweises auf die Patenterteilung: **28.07.1993**
(21) Anmeldenummer: 89106153.3
(22) Anmeldetag: 07.04.1989
(51) Int. Cl.: C07D 333/38

(54) **Verfahren zur Herstellung von 5-Chlor-3-chlorsulfonyl-2-thiophencarbonsäureestern**
Process for the preparation of 5-chloro-3-chlorosulphonyl-2-thiophene-carboxylic esters
Procédé de préparation d'esters d'acide 5-chloro-3-chloro-sulfonyl-2-thiophène carboxylique

(30) Priorität: 02.05.1988 AT 1123/88
(43) Veröffentlichungstag der Anmeldung: 08.11.1989
(73) Patentinhaber: Hafslund Nycomed Pharma Aktiengesellschaft, A-4021 Linz (AT)
(72) Erfinder: Wagner, Hans Peter, Dr., CH-4452 Itingen (CH)
(74) Vertreter: Kunz, Ekkehard, Dr.

(56) Entgegenhaltungen:
- EP-A- 0 001 113
- EP-A- 0 245 835
- GB-A- 2 159 156
- METHODEN DER ORGANISCHEN CHEMIE, (Houben-Weyl), 4. Auflage, Band V/3, 1962, Seiten 722-723, herausgegeben von E. Müller, Georg Thieme Verlag, Stuttgart, DE; E. FORCHE et al.: "Halogen-Verbindungen. Fluorverbindungen. Chlorverbindungen"

## Beschreibung

Die Erfindung betrifft ein Verfahren zur Herstellung von 5-Chlor-3-chlorsulfonyl-2-thiophencarbonsäureestern.

5-Chlor-3-chlorsulfonyl-2-thiophencarbonsäurealkylester (5-CCT) sind Zwischenprodukte bei der Herstellung pharmazeutisch wirksamer Substanzen. So werden beispielsweise in der US-PS 4,801,591 blutfettsenkende Substanzen beschrieben, bei deren Herstellung von 5-CCT ausgegangen wird. Auch die Darstellung von Chlortenoxicam (6-Chlor-4-hydroxy-2-methyl-3-(2-pyridyl-carbamoyl)-2H-thieno(2,3-e)1,2-thiazin-1,1-dioxid), einem Antirheumatikum, das in der US-PS 4,180,662 beschrieben ist, kann aus 5-CCT erfolgen.

In der GB-A 2 159 156 wird ein Verfahren zur Herstellung von mit Methyl oder Halogen substituierten 3-Chlorsulfonyl-2-thiophencarbonsäurealkylestern beschrieben, wobei die nur schwer zugänglichen 5-Chlor-3-amino-2-thiophencarbonsäurealkylester diazotiert und die gebildeten Diazoniumchloride anschließend mit SO₂ zu den Sulfochloriden umgesetzt werden. Dieses Verfahren ist jedoch umständlich und liefert nur ungenügende Ausbeuten.
Aus EP-A-0 245 835 ist unter anderen die Halogenierung von 2-(alpha-Alkoxyimino)ethylthiophenen bekannt, wobei diese Verbindungen jedoch mit dem (Alkoxyimino)ethylrest einen die Halogenierung erleichternden Elektronendonator als Substituent aufweisen.

Gegenstand der vorliegenden Erfindung ist ein Verfahren zur Herstellung von 5-Chlor-3-chlorsulfonyl-2-thiophencarbonsäureestern der Formel 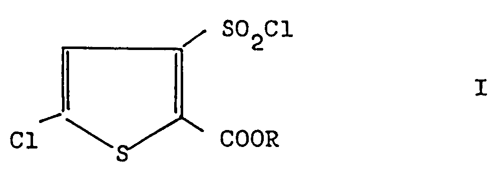
in der R C₁-C₄-Alkyl bedeutet,
dadurch gekennzeichnet,
daß man eine Verbindung der Formel 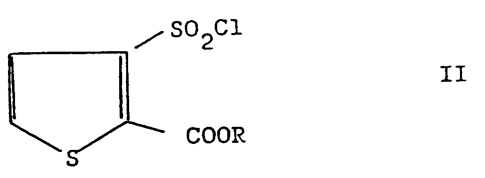
in der R die obige Bedeutung besitzt, in Gegenwart von aktiviertem Eisen durch Einleiten von Chlorgas chloriert.

Eine Methode zur Aktivierung des Eisens besteht darin, daß man pro Mol Verbindung II 0,1 bis 1,0 Mol, vorzugsweise 0,2 - 0,4 Mol metallisches Eisen in Pulver-oder Spanform in 0,5 - 5 l, vorzugsweise in 1 - 3 l organischem Lösungsmittel, das unter Reaktionsbedingungen inert ist, wie beispielsweise Methylenchlorid, Tetrachlorkohlenstoff oder in Mischungen solcher Lösungsmittel, suspendiert, wobei Methylenchlorid bevorzugt ist. Das Eisen wird durch Einleiten von ca. 100 - 500 g, bevorzugt von etwa 200 - 300 g Chlorgas pro Mol Eisen, aktiviert. Das Einleiten des Chlorgases erfolgt unter kräftigem Rühren der Eisensuspension innerhalb von 1 - 5, vorzugsweise von 2 - 3 Stunden bei einer Temperatur von etwa 10 - 50 °C, vorzugsweise 24 - 28 °C.

Eine weitere Methode der Aktivierung besteht darin, etwa gleiche Mengen von Eisen, wie oben beschrieben, im Reaktionskolben vorzulegen und 12 bis 48 Stunden lang, bevorzugt 24 Stunden lang, unter einer Chlorgasatmosphäre stehen zu lassen. Die Aktivierung des Eisens in einer Lösungsmittelsuspension ist jedoch bevorzugt.

Zur Chlorierung der 3-Chlorsulfonyl-2-thiophencarbonsäurealkylester (CT) löst man, falls die Aktivierung des Eisens in einer Lösungsmittelsuspension erfolgte, CT im gleichen Lösungsmittel oder Lösungsmittelgemisch, in dem das metallische Eisen suspendiert wurde, und zwar in etwa 0,3 - 5 l Lösungsmittel pro Mol CT, vorzugsweise in 0,5 bis 1 l Lösungsmittel pro Mol CT und vermischt diese Lösung rasch mit der Eisensuspension. Die Chlorierung von CT erfolgt unter Rühren durch Einleiten von ca. 5 - 50 g Chlorgas pro Stunde und Mol CT, bevorzugt von 15 -35 g pro Stunde und Mol CT, bei einer Temperatur von etwa 20 bis 50 °C, bevozugt bei einer Temperatur von 30 bis 32 °C. Der Reaktionsverlauf wird hiebei analytisch, bevorzugt durch Gaschromatographie, verfolgt. Nach Bildung von 50 - 70 %, bevorzugt 62 - 65 % Monochlorverbindung wird das Reaktionsgemisch auf Eiswasser gegossen und die Phasen werden getrennt. Die organische Phase wird getrocknet und eingedampft.

Falls die Aktivierung des Eisens unter einer Chlorgasatmosphäre erfolgte, wird CT vorzugsweise in 2 - 4 l einer der oben angeführten Lösungsmittel oder Lösungsmittelgemische gelöst, der Verlauf der weiteren Chlorierung ist wie oben beschrieben. Die Gesamtmenge des verwendeten Lösungsmittels ist bei beiden Möglichkeiten der Aktivierung gleich.

Die Reinigung des rohen 5-CCT kann nach üblichen Arten wie Umkristallisation, Säulen- und Verteilungschromatographie, Extraktion u.ä. erfolgen. Bevorzugterweise wird aus Diisopropylether umkristallisiert.

Die Ausgangsverbindung der Chlorierung, CT, ist literaturbekannt. Ihre Herstellung ist beispielsweise in der US-PS 4,028,373 beschrieben.

### Beispiel

### 5-Chlor-3-Chlorsulfonylthiophen-2-carbonsäuremethylester

In einem 20 l Vierhalskolben wurden 96 g Eisenpulver (1,71 Mol) (Baker, durch H₂ reduziert, mind. 96 %) in 12 l abs. Methylenchlorid suspendiert. Unter kräftigem Rühren wurden innerhalb von 2 bis 3 Stunden 440 g Chlorgas eingeleitet, wobei die Temperatur zwischen 24 und 28 °C betrug. Anschließend wurden 1,44 kg (5,98 Mol) 3-Chlorsulfonylthiophen-carbonsäuremethylester in 5 l abs. Methylenchlorid gelöst und rasch zugegeben. Unter Rühren wurden bei einer Temperatur von 30 bis 32 °C 100 bis 200 g Chlorgas pro Stunde eingeleitet, und der Reaktionsverlauf mittels Gaschromatographie verfolgt. Nach Bildung von 62 % bis 65 % Monochlorverbindung wurde das Reaktionsgemisch auf 24 l Eiswasser gegossen und während 15 min. kräftig gerührt. Nach Phasentrennung wurde die organische Phase getrocknet und der Rückstand bei 40 °C Badtemperatur im Vakuum eingedampft.

Der Rückstand wurde in 1,5 l Diisopropylether aufgenommen, filtriert und das Filtrat auf - 30 bis - 35 °C abgekühlt. Nach Animpfen mit Monochlorverbindung wurde ca. 15 bis 30 min. kristallisieren gelassen. Das Kristallisat wurde abgesaugt, mit 0,5 l Diisopropylether von - 30 °C gewaschen und im Vakuumschrank bei 25 °C getrocknet.
- Ausbeute:: 800 g Monochlorverbindung (48,7 %)
- GC:: 95 % Monochlorverbindung, Rest unchloriertes und dichloriertes Produkt.
- Fp:: 50 - 52 °C

## Patentansprüche

1. Verfahren zur Herstellung von 5-Chlor-3-chlorsulfonyl-2-thiophencarbonsäureestern der Formel 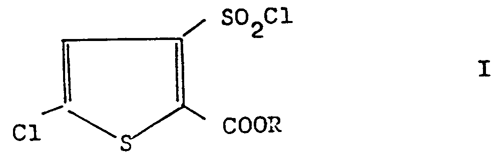 in der R C₁ - C₄-Alkyl bedeutet,
dadurch gekennzeichnet,
daß man metallisches Eisen mit Chlorgas aktiviert und eine Verbindung der Formel 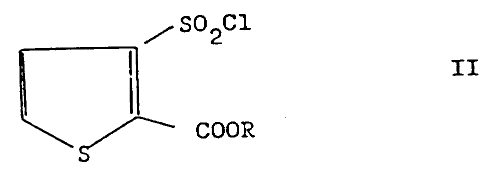 in der R die obige Bedeutung besitzt, in Gegenwart des aktivierten Eisens durch Einleiten von Chlorgas chloriert.

2. Verfahren nach Anspruch 1, dadurch gekennzeichnet, daß man das metallische Eisen unter einer Chlorgasatmosphäre mit Chlorgas aktiviert.

3. Verfahren nach einem der Ansprüche 1 oder 2, dadurch gekennzeichnet, daß man das metallische Eisen für etwa 12 bis 48 Stunden, bevorzugt für etwa 24 Stunden, unter einer Chlorgasatmosphäre aktiviert.

4. Verfahren nach Anspruch 1, dadurch gekennzeichnet, daß man das metallische Eisen in einer Lösungsmittelsuspension mit Chlorgas aktiviert, wobei pro Mol der Verbindung II 0,1 bis 1,0 Mol, bevorzugt 0,2 -0,4 Mol metallisches Eisen in 0,5 bis 5 l, vorzugsweise in 1 - 3 l unter Reaktionsbedingungen inertem organischen Lösungsmittel wie Methylenchlorid oder Tetrachlorkohlenstoff, oder in Mischungen solcher Lösungsmittel suspendiert und das Eisen durch Einleiten von 100 - 500 g Chlorgas pro Mol Eisen, bevorzugt von 200 - 300 g, während 1 - 5 Stunden, bevorzugt während 2 - 3 Stunden, aktiviert.

5. Verfahren nach einem der Ansprüche 1 bis 4, dadurch gekennzeichnet, daß man den Reaktionsverlauf der Chlorierung der Verbindung II analytisch, bevorzugt gaschromatographisch, verfolgt.

6. Verfahren nach einem der Ansprüche 1, 4 oder 5 , dadurch gekennzeichnet, daß man die Aktivierung des Eisens und die Chlorierung der Verbindung der Formel II im gleichen Lösungsmittel oder Lösungsmittelgemisch durchführt.

7. Verfahren nach einem der Ansprüche 1 bis 6, dadurch gekennzeichnet, daß man die Chlorierung der Verbindung der Formel II durch Einleiten von 5 - 50 g Chlorgas pro Stunde und Mol der Verbindung II, bevorzugt 15 - 35 g/Stunde und Mol Verbindung II, bei 20 bis 50 °C, bevorzugt bei 30 - 32 °C so lange durchführt, bis 50 - 70 %, bevorzugt 62 - 65 % Monochlorverbindung gebildet sind und das Reaktionsgemisch anschließend auf Eiswasser gießt.

## Claims

1. A process for the preparation of 5-chloro-3-chlorosulphonylthiophene-2-carboxylic acid esters of the formula 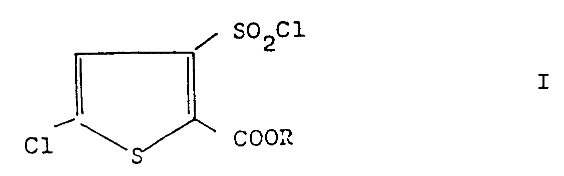 in which R is C₁-C₄-alkyl,
characterised in that metallic iron is activated with chlorine gas and a compound of the formula 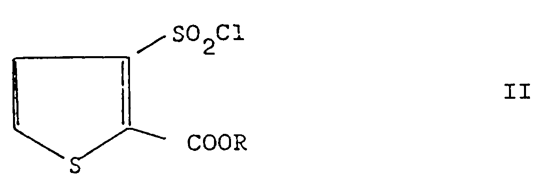 in which R is as defined above, is chlorinated by the introduction of chlorine gas in the presence of the activated iron.

2. A process according to Claim 1, characterised in that the metallic iron is activated with chlorine gas under a chlorine gas atmosphere.

3. A process according to Claim 1 or Claim 2, characterised in that the metallic iron is activated under a chlorine gas atmosphere for about 12 to 48 hours, preferably for about 24 hours.

4. A process according to Claim 1, characterised in that the metallic iron is activated with chlorine gas in a solvent suspension, 0.1 to 1.0 mol, preferably 0.2 - 0.4 mol of metallic iron, per mol of compound II, being suspended in 0.5 to 5 l, preferably in 1 - 3 l of an organic solvent which is inert under the reaction conditions, such as methylene chloride or carbon tetrachloride, or in mixtures of such solvents, and the iron being activated by the introduction of 100 - 500 g of chlorine gas per mol of iron, preferably of 200 - 300 g, for 1 - 5 hours, preferably for 2 - 3 hours.

5. A process according to one of Claims 1 to 4, characterised in that the course of the chlorination reaction of the compound II is monitored by analysis, preferably by gas chromatography.

6. A process according to one of Claims 1, 4 or 5, characterised in that the activation of the iron and the chlorination of the compound of formula II are carried out in the same solvent or solvent mixture.

7. A process according to one of Claims 1 to 6, characterised in that the compound of formula II is chlorinated by the introduction of 5 - 50 g of chlorine gas per hour and per mol of compound II, preferably of 15 - 35 g/hour per mol of compound II, at 20 to 50°C, preferably at 30 - 32°C, until 50 - 70%, preferably 62 - 65% of the monochloro compound has been formed, and the reaction mixture is then poured on to iced water.

## Revendications

1. Procédé de préparation d'esters de l'acide 5-chloro-3-chlorosulfonyl-2-thiophène carboxylique, de formule : 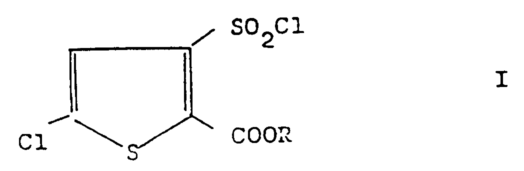 dans laquelle R signifie un groupe alkyle de 1 à 4 atomes de carbone, caractérisé en ce qu'on active du fer métallique avec du chlore gazeux et on réalise la chloruration d'un composé de formule : 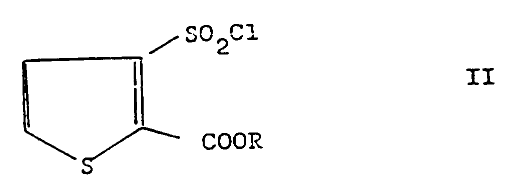 dans laquelle R a la signification sus-indiquée en présence du fer activé par introduction de chlore gazeux.

2. Procédé selon la revendication 1, caractérisé en ce qu'on active le fer métallique sous atmosphère de chlore gazeux avec du chlore gazeux.

3. Procédé selon l'une des revendications 1 ou 2, caractérisé en ce que le fer métallique est activé sous atmosphère de chlore gazeux durant environ 12 à 48 heures, avantageusement pendant 24 heures.

4. Procédé selon la revendication 1, caractérisé en ce qu'on active le fer métallique dans une suspension de solvant avec du chlore gazeux,en mettant en suspension par mole de composé II 0,1 à 1,0 mole, avantageusement 0,2 à 0,4 mole, de fer métallique dans 0,5 à 5 l de solvants organiques inertes dans les conditions de la réaction, avantageusement dans 1 à 3 l, comme le chlorure de méthylène, ou le tétrachlorure de carbone, ou des mélanges de ces solvants, et on active le fer par introduction de 100 à 500 g de chlore gazeux par mole de fer, avantageusement de 200 à 300 g, durant 1 à 5 heures, avantageusement pendant 2 à 3 heures.

5. Procédé selon l'une des revendications 1 à 4, carctérisé en ce qu'on suit le déroulement de la réaction de chloruration du composé de manière analytique, avantageusement par chromatographie en phase gazeuse.

6. Procédé selon l'une des revendications 1,4 ou 5, caractérisé en ce que l'activation du fer et la chloruration du composé de formule II sont conduits dans le même solvant ou mélange de solvants.

7. Procédé selon l'une des revendications 1 à 6, caractérisé en ce qu'on réalise la chloruration du composé de formule II par introduction de 5 à 50 g de chlore gazeux par heure et par mole du composé II, avantageusement 15 à 35 g par heure et mole de composé II, de 20 à 50°C, avantageusement de 30 à 32°C, jusqu'à la formation de 50 à 70% de composé monochloré, avantageusement de 62 à 65%, et on verse ensuite le mélange réactionnel sur de l'eau glacée.
